# EUROPEAN PATENT APPLICATION

(11) **EP 2 954 929 A1**
(43) Date of publication of application: **16.12.2015**
(21) Application number: 15168298.6
(22) Date of filing: 20.05.2015
(51) Int. Cl.: A61N 1/375

(54) **LEAD CONNECTOR ASSEMBLY, MEDICAL DEVICE AND MANUFACTURING METHOD FOR SAME**

(30) Priority: 10.06.2014 US 201462009926 P
(71) Applicant: BIOTRONIK SE & Co. KG, 12359 Berlin (DE)
(72) Inventor: Brunner, Bjoern, Portland, OR Oregon 97219 (US); Roos, John, Sherwood, OR Oregon 97140 (US)
(74) Representative: Galander, Marcus

(57) **Abstract**

A lead connector assembly having an open and a closed end, and one or more electrically conducting contact rings spaced apart by at least one electrically insulating ring forming a ring stack. Each contact ring includes an electrically conducting pin protruding from the respective contact ring, and an electrically insulating end ring accommodated at the end of the ring stack at the open end. The inner surfaces of: the one or more contact rings; the at least one insulating ring; and the end ring, define a lead aperture starting at the open end. Connector housing parts enclose the contact rings, the at least one insulating ring and the end ring, and form an outer surface and are joined such that a hermetic seal is provided between the lead aperture and the outer surface. The electrically conducting pin of each contact ring projects through the joined connector housing parts and from the outer surface.

## Description

The present invention relates to a lead connector assembly for a medical device, the lead connector assembly having an open end and a closed end and comprising one or more electrically conducting contact rings and, if applicable, spaced apart by at least one electrically insulating and/or sealing ring forming a ring stack, wherein each contact ring comprises an electrically conducting pin protruding from the respective contact ring and an electrical insulating end ring accommodated at the end of the ring stack at the open end of the lead connector assembly, wherein the inner surface of each of the one or more contact rings, of each of the at least one insulating and/or sealing ring and of the end ring define a lead aperture starting at the open end of the lead connector assembly. The invention further relates to a medical device comprising a power source connected with an electronic circuit having a circuit board and a manufacturing method for such a lead connector assembly and for such a medical device.

Implantable medical devices for providing electrical stimulation to body tissues, for monitoring physiologic conditions, and for providing alternative treatments to drugs are well-known in the art. Exemplary implantable medical devices include implantable cardio defibrillators, pace makers, and programmable neuro-stimulator pulse generators. The medical devices typically incorporate a power source connected with an electronic circuit having a circuit board. Connected to the sealed housing often a header assembly is provided which includes electrical contact elements that are electrically coupled with the electronic circuit and/or to the power source located inside the hermetically sealed device housing via a feedthrough component. The header assembly provides a connector means for electrically communicating via an external medical lead cable. The current implantable devices utilize a discrete feedthrough component to isolate the electronic circuit and the power source (battery) from body fluid intrusion. This requires that electrical connections be made between the circuit board and each feedthrough pin, between each feedthrough pin and a header ribbon, and between the header ribbon and each connector terminal. Creating three (or more) weld connections for each signal adds costs and has the potential to reduce the reliability of the module. Typical implantable medical devices use a circuit board ribbon, a feedthrough, and a header ribbon connection between the electronic circuit and the lead to provide hermetic sealing and strain relief functions to the electronic circuit.

Document US 7,711,428 B2 discloses an implantable medical device with a hermetic lead connector assembly for such a device. The lead connector of the lead connector assembly includes one or more electrically conducting contact rings spaced apart by one or more electrically insulating rings. Further, a rigid sleeve is disposed about the hermetic lead connector outer surface. The rigid sleeve has an aperture that exposes a portion of the one or more electrically conducting contact rings and pins projecting therefrom and aids in reducing a bending moment along the length of the lead connector. Further, the electrically insulating ring is brazed between two electrically conducting contact rings and/or the electrically conducting spacer ring. The braze produces the hermetic seal between the sealed housing interior/lead connector outer surface and the lead aperture.

Document US 8,131,370 B2 refers to a method of manufacturing a hermetic lead connector including fixing an electrically insulating ring between an electrically conducting contact ring and an electrically conducting spacer ring to form a hermetic ring subassembly, and fixing a plurality of hermetic ring subassemblies in axial alignment to form a hermetic lead connector. This hermetic lead connector provides a hermetic seal between the outer surface and the inner surface. Further, a mounting flange is provided which is fixed to the known lead connector adjacent the open end. The mounting flange can be brazed or welded to the hermetically sealed housing. Each electrically conducting contact ring comprises a pin for electrical communication with the electronics.

Documents US 8,131,370 B2 and US 7,711,428 B2 describe a sealed connector composed of alternating rings made from electrically conducting contact ring and electrically isolating spacer ring which require a braze joint be formed between each of these rings to provide a hermetic seal which is very costly in production.

Document US 8,328,587 B2 refers to a connector assembly for use with implantable medical devices disclosing easy to assemble contacts. The document discloses an in-line connector stack placed inside an encapsulation layer so that the encapsulated stack may be tested for aligning and conductivity before it is installed or placed into a header of a medical device. In particular, a connector stack which comprises a plurality of seal elements, conductive elements and spring contact elements are configured to fit inside an encapsulation layer to form an encapsulated stack. The encapsulated stack is configured to fit inside a molded header which has slots or openings for accessing to weld the leads from the electronic case with the conductive elements.

The above-mentioned existing implantable medical devices include up to seven electrical material junctions between each lead cable contact and the circuit board. The typical device has the following interfaces: a solder joint connection between the circuit board and a weld pad, a weld connection between the weld pad and a weld ribbon, a weld connection between the weld ribbon and a feedthrough pin, a weld connection between the feedthrough pin and a header ribbon, a weld connection between the header ribbon and a connector ring, a mechanical connection between the connector ring and a connector spring, and a mechanical connection between the connector spring and the lead. Each of these connections requires time and material to create and introduce the opportunity for intermittent connections during use.

Hence, it is an object of the present invention to connect the electronic circuit to the removable lead connector of a medical device with minimum cost and maximum reliability. Further, moisture intrusion from the body into the electronic circuit and power source needs to be prevented in order to avoid electrical shorts and leaks.

The above task is solved by a lead connector assembly further comprising at least two connector housing parts which enclose the one or more contact rings, the insulating and/or sealing ring, and the end ring and forming an outer surface of the lead connector assembly, wherein the connector housing parts are joined such that a hermetic seal is provided between the lead aperture and the outer surface of the lead connector assembly, wherein the electrically conducting pin of each contact ring projects through at least one of the joined connector housing parts and from the outer housing of the lead connector assembly.

The above inventive lead connector assembly uses the finding of the inventors that each material junction in the path between the electric circuit board and the lead enhances the likelihood of moisture intrusion and electrical shorts and leaks. Hence, the invention is based on the idea to minimize the total connection count.

The hermetic seal is provided by the at least two connector housing parts, preferably two or three connector housing parts which enclose the one or more contact rings, the electrically insulating and/or sealing ring, if applicable, and the end ring. The rings do not need to be hermetically sealed so that only the intrusion-tight joining of the connector housing parts is to be manufactured, for example, by welding. Accordingly, the manufacturing costs are reduced without losing the reliability of the lead connector assembly.

In case that at least two connector housing parts are formed by metal parts which are easy to manufacture and weld, for example by seam welding, an electrically insulating layer is accommodated between the outer surface of the one or more contact rings and the at least two connector housing parts, for example by inserting two insulating sleeves into the respective opening between the outer surface of the one or more contact rings and the at least two connector housing parts or by injection molding or by casting. Preferably, the insulating layer also encloses the electrically insulating and/or sealing ring, if applicable, and the end ring.

Further, an insulating bar as a connector housing part is provided having openings for projection of the electrically conducting pin of each contact ring. The insulating bar encloses the end of each pin located opposite the respective contact ring. Further, the insulating bar comprises a braze pre-form around its outside. The insulating bar may be placed into a respective opening of another connector housing part (lower housing part) in order to connect it to the other connector housing part forming the connector housing. After accommodation of the electrically conducting pin of each contact ring into a respective opening of the insulating bar the insulating bar is brazed to the other housing part and to the pins of the contact rings. Preferably, for correct alignment a ring alignment stylus is provided.

For reliable attachment of the insulating bar to the other connector housing part this connector housing part may provide a housing flange surrounding the opening for the insulating bar. This housing flange may be stamped integrally with the respective connector housing part or it may be seam welded or brazed to the respective lathe turned other connector housing part.

In a preferred embodiment the one or more contact ring forms a spring contact, in particular a garter spring contact by providing a garter spring in a respective cavity of the contact ring.

In another embodiment a set screw block is provided which is accommodated at the end of the ring stack and at the closed end of the lead connector assembly and enclosed by the at least two connector housing parts. This set screw block configuration is used for reliably securing the lead connectors of the standard DF4 medical lead to the lead connector assembly (and therefore for reliably fixing it to the medical device) and allows for compliance with the DF4 industry standard. This set screw block can further be welded directly to the device housing to provide the case ground connection in lieu of a case ground pin which is often incorporated into discrete feedthroughs.

The above task is further solved by above mentioned medical device comprising at least one of the above described lead connector assemblies, wherein each pin projecting from the outer surface of each lead connector assembly is directly electrically connected to a terminal of the electronic circuit.

Therein each pin may be plated with a solderable interface material, or discrete solder pads may be brazed to their ends in order to form the connection to the respective terminal of the electronic circuit of the medical device. The pin may be wire bonded, welded, or thermo-compression bonded to the respective terminal of the electronic circuit. Further, the pin may be formed round or oblong, wherein a plurality of pins may form an in-line or offset pattern to increase connector stability through the reflow solder process.

Forming a direct connection to a terminal of the electronic circuit means that a pin is directly soldered, wire bonded, welded, or thermo-compression bonded to the respective terminal at the surface of the circuit board without any intermediate element like a ribbon. This design eliminates all weld ribbons, feedthrough pins, and header ribbons from the device including all interconnections between them. Preferably, the solder joints are created through automatic pick and place solder reflow processes which eliminates numerous manual steps from the device assembly process including the potential for quality defects and scrap created by these operations.

In a preferred embodiment each terminal for connection with the respective pin of the lead connector assembly is provided at a flexible arm of the circuit board. This has the advantage that the strain relief function previously provided by the weld ribbons is now incorporated into the flex arm of the circuit board to prevent excessive stress on any of the interfaces. Accordingly, the inventive medical device can be manufactured at reduced cost and with increased reliability compared to the known medical devices.

In another preferred embodiment the circuit board may be made from rigid FR4 or alumina.

In another embodiment further a device housing is provided enclosing the power source, the electronic circuit and the at least one lead connector assembly, preferably such that the lead aperture is still accessible from the outside so that a connector plug can be placed in the aperture. Preferably, the device housing comprises two halves which are seam welded to form a hermetically sealed enclosure. This creates a medical device where the inside of the connector with all solder joint interfaces are protected from fluid intrusion by the device housing. In such a medical device the lead aperture is the only portion exposed to the outside of the medical device, and, if applicable, the interior of the chimney and the welding ring of the set screw block.

In order to make the individual contact rings less expensive to machine or lathe, in one embodiment of the lead connector assembly the conducting pins may be welded or brazed to the contact rings.

In order to ease and cheapen the manufacturing process a solder reflow temperature resistant garter spring contact and a silicone seal may be used so that the lead connector assembly may be fully assembled and welded prior to soldering it to the circuit board of the electronic circuit.

For increasing signal count using standard DF4 lead connectors the inventive medical device may comprise two or more lead connector assemblies connected to terminals preferably provided at flex arms of a circuit board.

Further, numerous contact rings may be added to each lead connector assembly if more signals are required per lead.

In another embodiment, one of the connector signals may be routed outside of the medical device for use as a radio frequency communication antenna or a solder pin may be added to the end ring and a wire routed from there to an antenna placed on the outside of the medical device.

The inventive method of manufacturing the lead connector assembly solving the above objective comprises the following steps:
a) inserting the one or more electrically conducting contact rings with their respective pin into an according opening in the at least one first connector housing part,
b) hermetically sealed joining the contact rings with the at least one first connector housing part,
c) accommodating at least one electrically insulating and/or sealing ring between two contact rings, if applicable, and accommodating an end ring at the end of the ring stack at the open end of the lead connector assembly, and
d) hermetically sealed joining of at least a second connector housing part to the first connector housing part.

The inventive method is cost effective and provides a reliable lead connector assembly.

Regarding step a) the at least one first connector housing part is preferably formed as an insulator bar, most preferably made of ceramic, which is in a further preferred embodiment inserted into a respective opening of another connector housing part (lower housing part) along with a braze pre-form around its outside. Further, the contact rings are inserted into holes in the insulator bar along with a braze pre-form.

Preferably, in case the connector housing is mainly made of a current conducting material such as metal, prior step d) in step d1) at least one electrically insulating layer is accommodated between the outer surface of the one or more contact rings and the inner surface of the at least one first connector housing part, for example by rotating two halves of a sleeve into the respective opening or by injection molding or by casting.

In another preferred embodiment of the inventive method, after insertion of a ring alignment stylus through the lead aperture in each of the contact rings, the assembly, preferably comprising the insulator bar, the other connector housing part, the contact rings and the set screw block is brazed together in an oven. After that in another preferred embodiment an SMT pick feature is clipped into place.

In another embodiment, a set screw block is provided comprising a block insulator and chimney which are stacked on top of the set screw block along with braze pre-forms to insulate the set screw block from the connector housing part.

In a further embodiment of the inventive method of manufacturing a lead connector assembly, prior to step d) and d1) the set screw block is accommodated at the end of the ring stack located at the closed end of the lead connector assembly.

The above task is further solved by a method of manufacturing the above described medical device comprising the above mentioned steps of the method of manufacturing the lead connector assembly and the additional steps
e) directly electrically connecting each pin projecting from the outer surface of each lead connector assembly to a terminal of the electronic circuit and
f) enclosing the power source, the electronic circuit and the at least one lead connector assembly with a device housing.

A full and enabling disclosure of the present invention, including the best mode thereof, directed to one of ordinary skill in the art is set forth in the following specification of the preferred embodiments. Thereby, further features and advantages are presented that are part of the present invention independently of the features mentioned in the dependent claims.

The specification refers to the accompanying figures showing schematically
- Fig. 1: a first embodiment of an inventive medical device in a perspective side view,
- Fig. 2: the inventive medical device of Fig. 1 with a removed device housing part in a perspective side view,
- Fig. 3: an inventive lead connector assembly in an exploded view from the side,
- Fig. 4: a cross section of the inventive lead connector assembly of Fig. 3 with a section of the inventive medical device of Fig. 1 in a perspective side view,
- Fig. 5: a first assembly step of an inventive method of manufacturing the medical device of Fig. 1 in a perspective side view,
- Fig. 6: a second step of the inventive method started in Fig. 5 in a perspective side view,
- Fig. 7: a third step of the inventive method started in Fig. 5 in a perspective side view,
- Fig. 8: a fourth step of the inventive method started in Fig. 5 in a perspective side view,
- Fig. 9: a fifth step of the inventive method started in Fig. 5 in a perspective side view,
- Fig. 10a: sixth step of the inventive method started in Fig. 5 in a perspective side view,
- Fig. 11: a second embodiment of an inventive medical device in a perspective side view with one housing part removed,
- Fig. 12: a third embodiment of an inventive medical device in a perspective side view with one part of the device housing removed and
- Fig. 13: a process chart comprising the inventive method of Fig. 5 to 10.

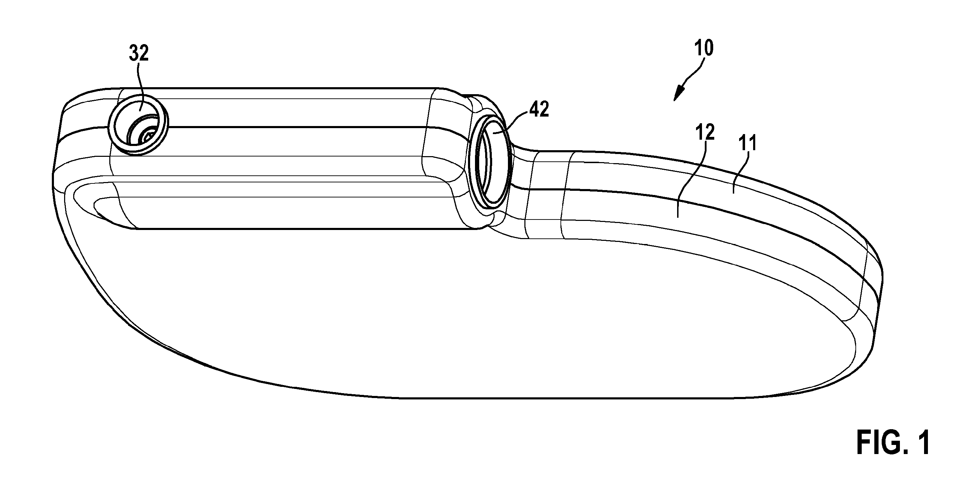

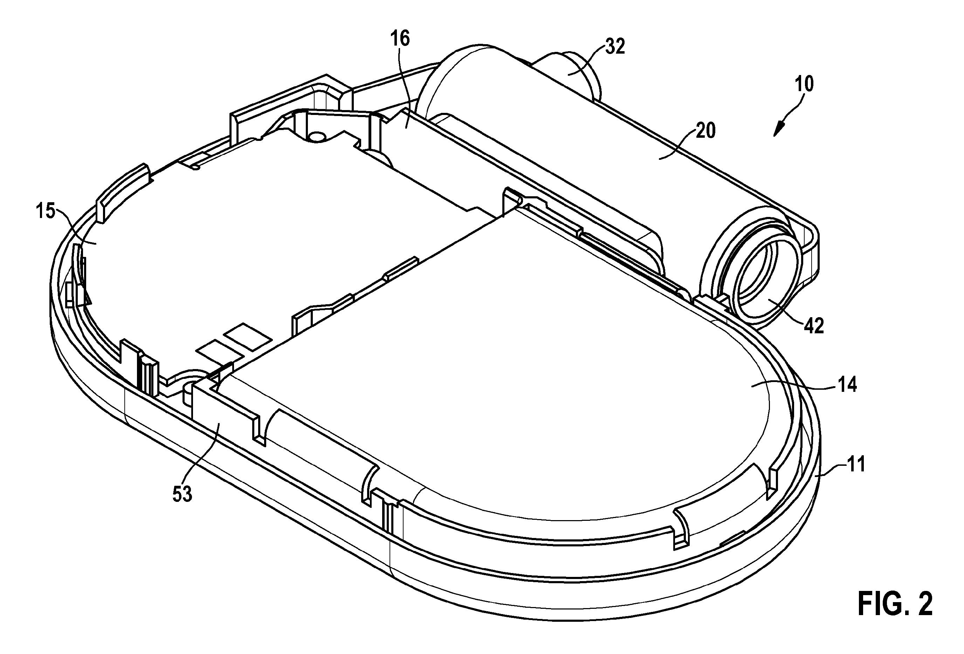

The first embodiment of an inventive medical device 10 as depicted in Fig. 1 and 2, for example an implantable pulse generator, comprises a first device housing part 11 and a second device housing part 12 which are joined by seam welding. Further, the medical device 10 comprises a power source 14 in form of a battery and an electronic circuit (not shown) provided on a circuit board 15. The electronic circuit is electrically connected to the power source 14.

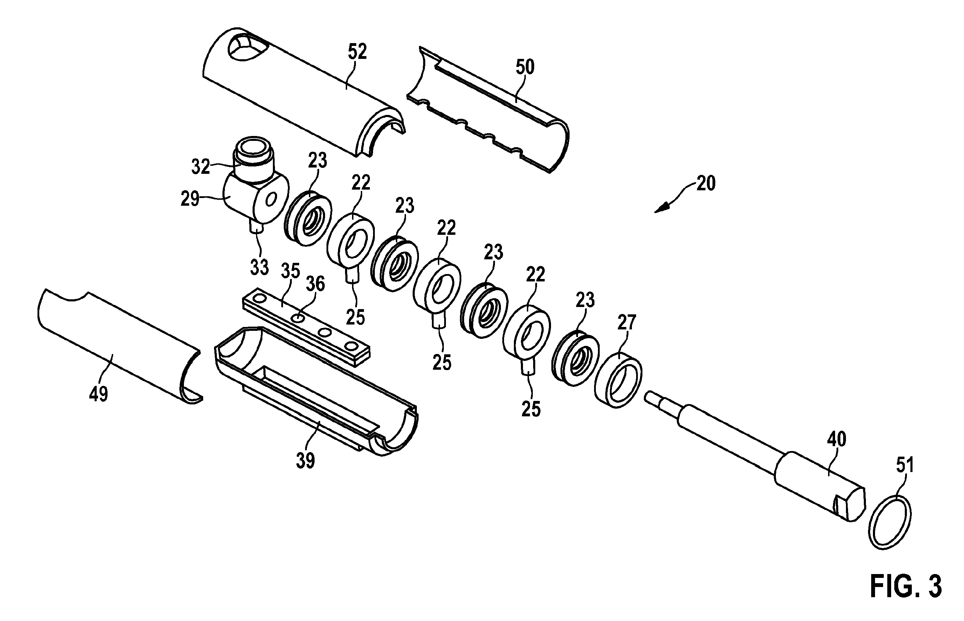

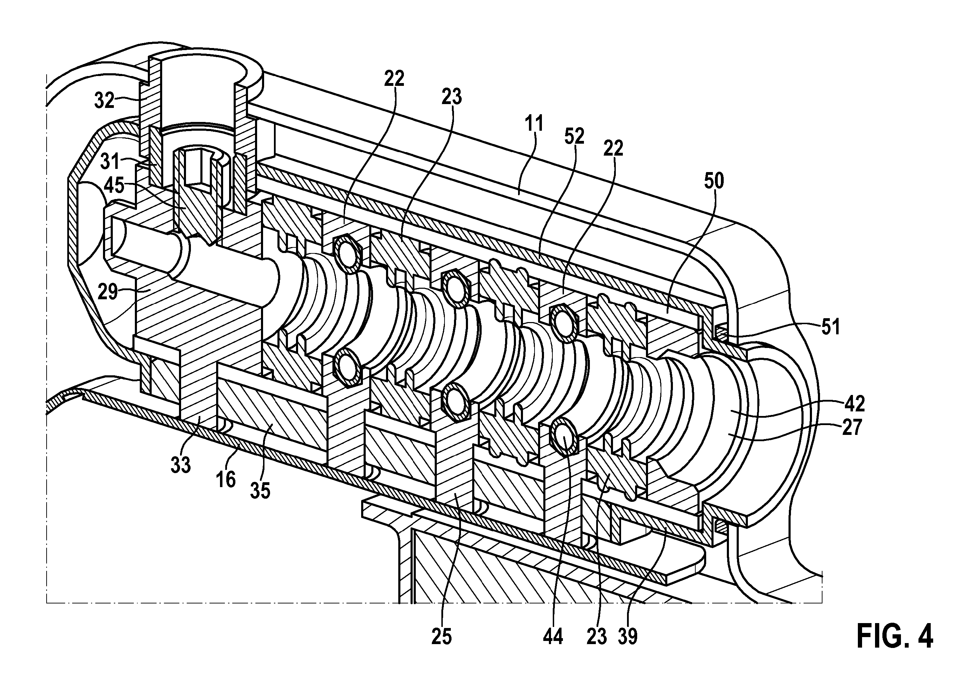

For connection to an external lead plug (not shown) a lead connector assembly 20 is provided with the components shown in Fig. 3 and 4 in detail. The lead connector assembly 20 comprises three contact rings (terminal rings) 22 spaced apart by electrically insulating and/or sealing rings 23. Each contact ring 22 comprises a pin 25 for electrical connection with the respective terminal of the electronic circuit provided on the circuit board 15.

At the open end of the lead connector assembly 20 an insulating end ring (end cap) 27 is provided. At the closed end of the lead connector assembly 20 a set screw block 29 is accommodated with an electrically insulating chimney 31 and a weldable ring 32. This set screw block 29 further comprises a pin 33 projecting from the lower side of the outer surface.

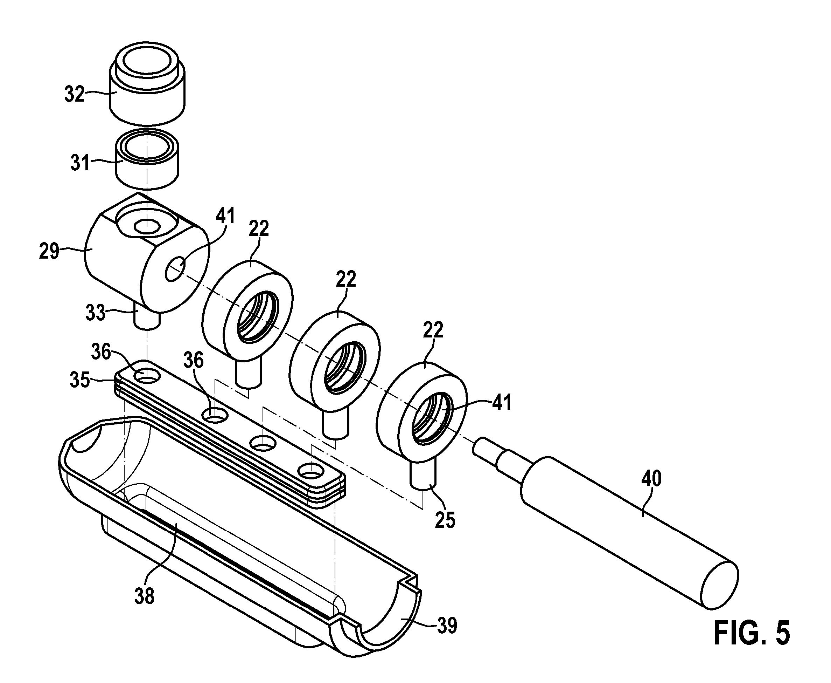

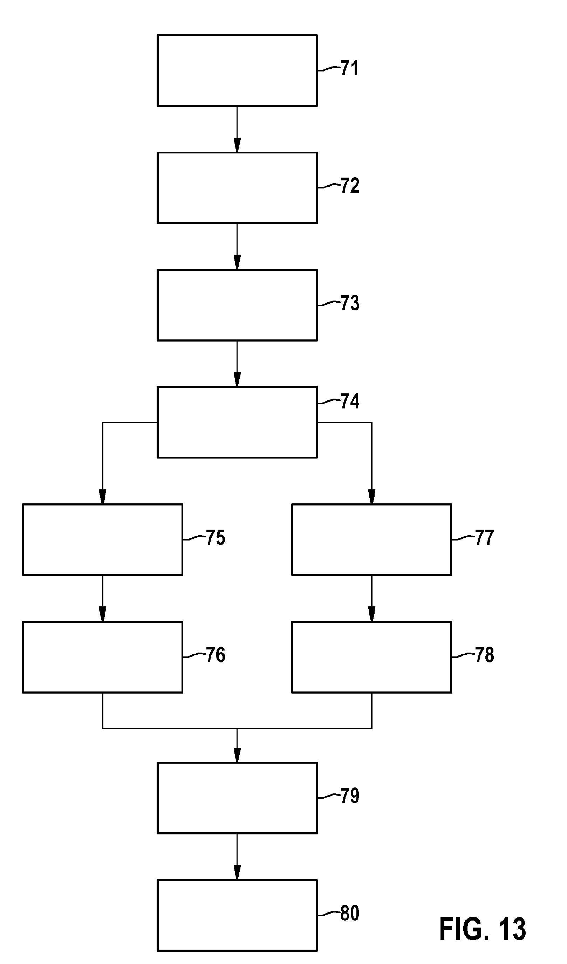

In order to manufacture the lead connector assembly 20 and the medical device in a first step 71 (see Fig. 5 and 13) a ceramic insulator bar 35 is provided comprising through-going openings 36. The insulator bar 35 is placed in a respective through-going opening 38 of a lower connector housing part 39 with a braze pre-form around its outside. Then as it is shown in Fig. 5 pins 25 of the contact rings 22 and the pin 33 of a set screw block 29 are inserted into the respective opening 36 of the insulator bar 35 along with a braze pre-form. Now, an insulating chimney 31 and a weldable ring 32 are stacked on top of the set screw block 29 along with braze pre-forms to isolate the chimney 31 from the connector housing.

Now, a ring alignment stylus 40, which is only a helping means for the manufacturing process and thus does not belong to the inventive lead connector assembly 20 or the inventive medical device 10, is inserted through the lead opening 41 in each of the contact rings 22 and in the set screw block 29 forming together the lead aperture 42. This assembly is then brazed together in an oven and a SMT pick feature 43 is clipped into place.

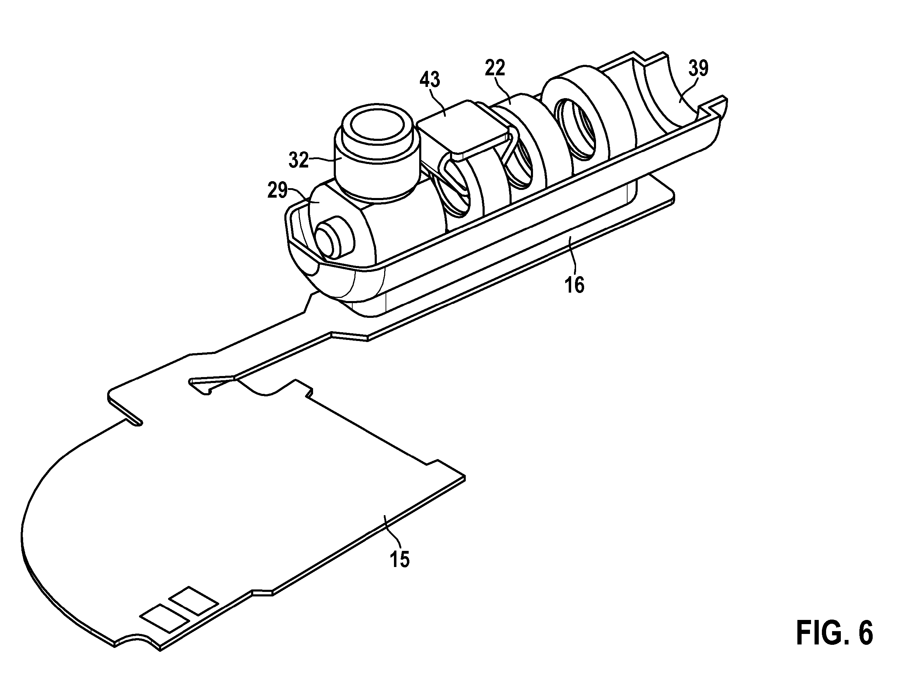

Now in step 72 (see Fig. 6 and 13), the pins 25, 33 of the contact rings 22 and the set screw block 29 are directly soldered to respective terminals (not shown) provided at a flexible arm 16 of the circuit board 15.

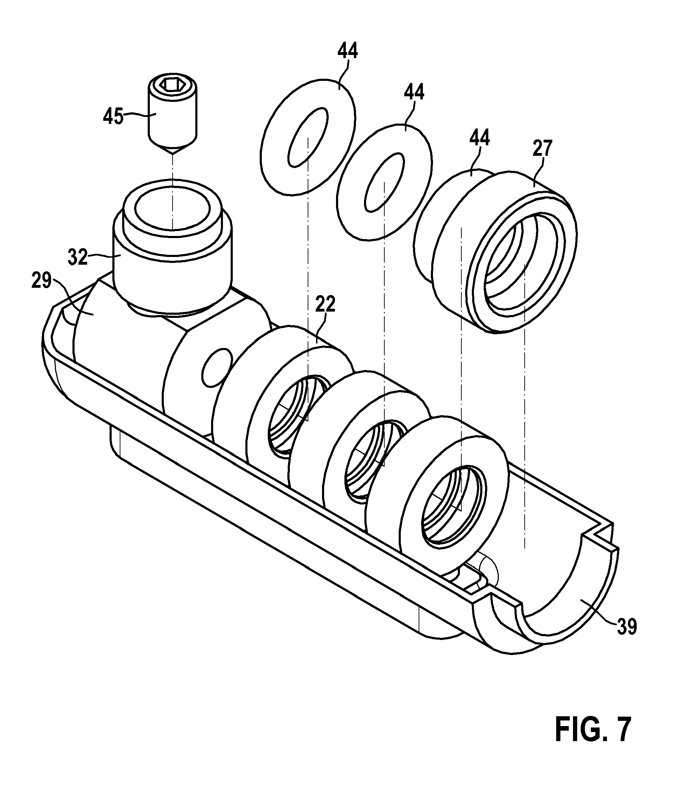

Now, as shown in Fig. 7 and indicated in step 73 of Fig. 13 the preferably polymer or ceramic end ring 27 is placed at the open end of the lead connector assembly into the lower connector housing part 39 and a garter spring 44 as a contact is placed within a respective cavity of each contact ring 22. Additionally, a conductive set screw 45 is placed within the chimney 31 of the set screw block 29.

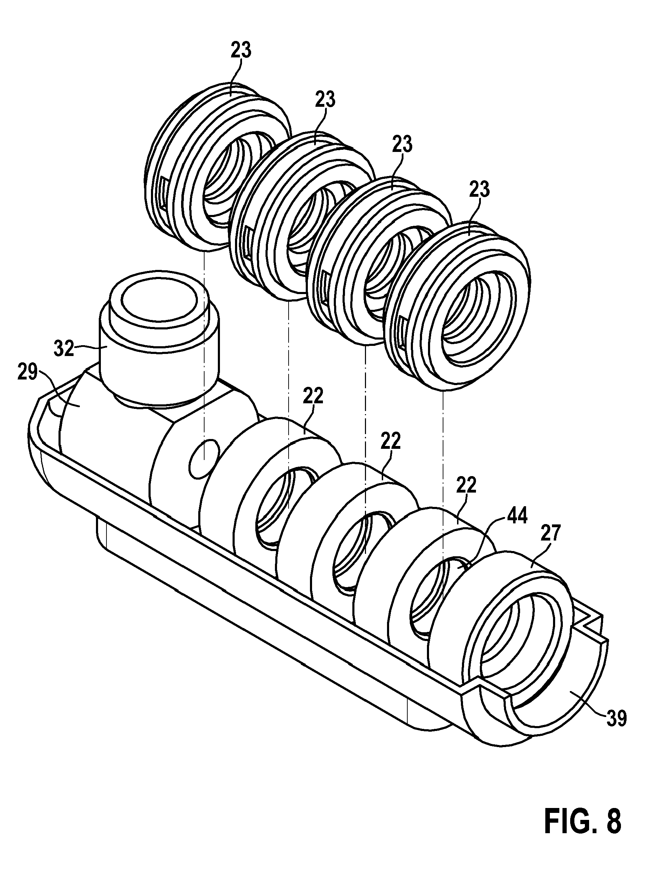

In the following step as shown in Fig. 8 and indicated in step 74 of Fig. 13, electrically insulating and/or sealing rings 23, preferably made of an elastomer material, are placed between the contact rings 22 and the set screw block 29 or the end ring 27 at the lower connector housing part 39 such that a ring stack is formed comprising the end ring 27, the contact rings 22 with garter spring 44 contact, the electrically insulating and/or sealing rings 23, and the set screw block 29.

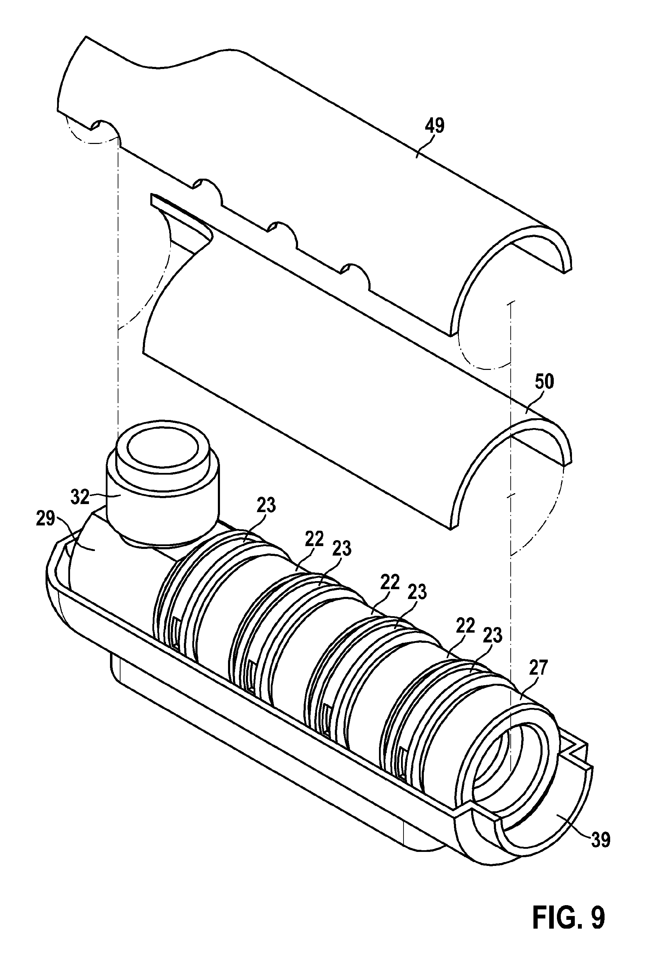

Then, as shown in Fig. 9 and indicated in step 75 of Fig. 13 a first insulating sleeve 49 and a second insulating sleeve 50, both preferably made of a polymer material, are provided and rotated into place into a cavity between the inner surface of the lower connector housing part 39 and the outer surface of the contact rings 22, the electrically insulating and/or sealing rings 23, the end ring 27, and the set screw block 29, respectively. The accommodation of the first insulating sleeve 49 and the second insulating sleeve 50 may also be derived from the cross section of the lead connector assembly 20 shown in Fig. 4. Thereby, the lower connector housing part 39 made of metal material is electrically isolated from the contact rings 22.

In the following step 76 of Fig. 13, an upper connector housing part 52 is provided and seam welded to the lower connector housing part 39 forming a hermetic seal.

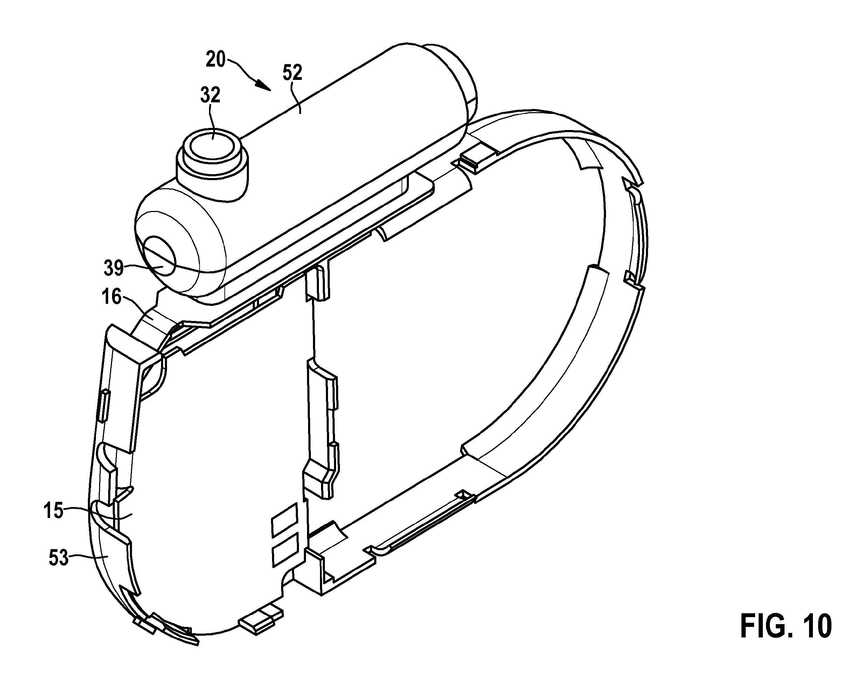

In the next step 79 (see Fig. 13) as depicted in Fig. 10 an insulating frame 53 of the medical device 10 is provided for carrying the circuit board 15 and the lead connector assembly 20. Then, the power source 14 is placed within the frame 53 and electrically connected to the respective terminals of the electronic circuit provided with the circuit board 15. Additionally, a weld spatter ring 51 is placed at the outer surface of the aperture 42 of the lead connector assembly 20. After this, the frame 53 with the above mentioned elements is accommodated within the first device housing part 11 (see Fig. 2).

Now in step 80 (see Fig. 13 and 1), the second device housing part 12 is provided and joined to the first device housing part 11 by seam welding providing a hermetic seal.

Alternatively, in replacement of steps 75 and 76 the steps 77 and 78 may be conducted after step 74. Therein, in step 77 the upper connector housing part 52 is seam welded to the lower connector housing part 39 with the above mentioned ring stack (step 77). Then, an insulating layer is injection molded into the cavity between the inner surface of the lower and upper connector housing part 39, 52 and the outer surface of the contact rings 22, the electrically insulating and/or sealing rings 23, the end ring 27 and the set screw block 29, respectively, around the pins 25, 33 (step 78).

In summary, the electric connection between a terminal of the electronic circuit provided on the circuit board 15 to the lead plug is facilitated by the pin 25 of the contact ring 22, the contact ring 22 and the garter spring 44.

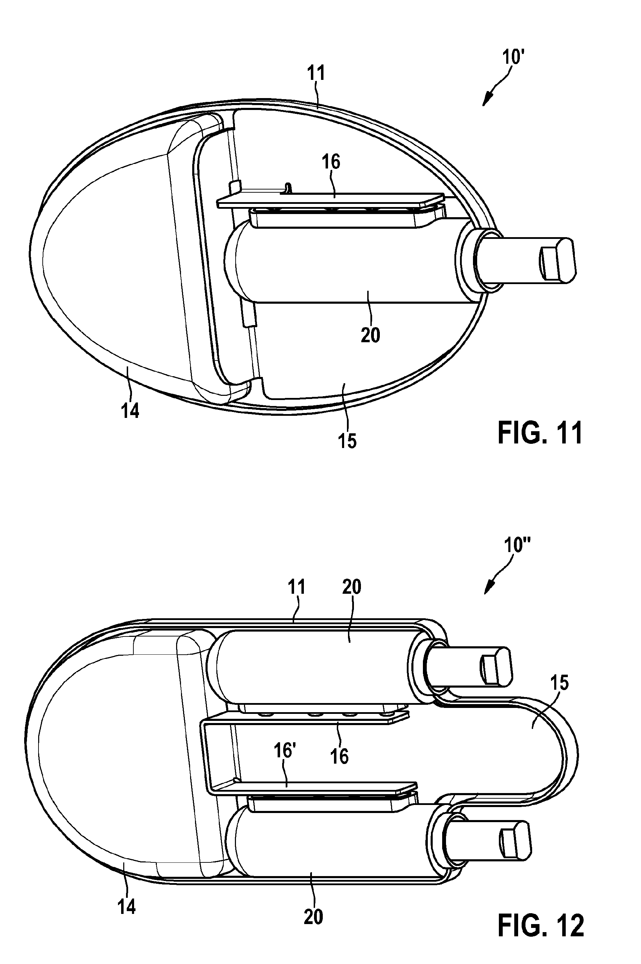

Fig. 11 shows a second embodiment of an inventive medical device 10' wherein the lead connector assembly 20 is situated near the center line of the device. In contrast to this the lead connector assembly of the first embodiment of the medical device as shown in Fig. 1 and 2 is provided at one longer side of the device. Fig. 12 discloses a third embodiment of an inventive medical device 10" comprising two lead connector assemblies 20 wherein the pins of each lead connector assembly 20 are connected to terminals of another flexible arm 16, 16' of the circuit board 15. Thereby signal count may be increased even if standard DF4 lead connectors are used.

It shall be emphasized again that the inventive hermetic sealed lead connector assembly 20 and the inventive hermetic sealed medical device 10, 10', 10" eliminate all weld ribbons, feedthrough pins, and header ribbons including all interconnections between them. The strain relief function previously provided by the weld ribbons is incorporated into the flex arm 16, 16' of the circuit board 15 to prevent excessive stress on any of the interfaces. With the inventive method for manufacturing costs are reduced and reliability of devices is increased.

### List of reference signs

- 10, 10', 10": medical device
- 11: first device housing part
- 12: second device housing part
- 14: power source
- 15: circuit board
- 16, 16': flexible arm
- 20: lead connector assembly
- 22: contact ring
- 23: electrically insulating and/or sealing ring
- 25: pin
- 27: end ring
- 29: set screw block
- 31: chimney
- 32: weldable ring
- 33: pin
- 35: insulator bar
- 36: opening in insulator bar 35
- 38: opening in lower connector housing part 39
- 39: lower connector housing part
- 40: alignment stylus
- 41: lead opening
- 42: lead aperture
- 43: SMT pick feature
- 44: garter spring
- 45: set screw
- 49: first insulating sleeve
- 50: second insulating sleeve
- 51: weld spatter ring
- 52: upper connector housing part
- 53: insulating frame
- 71, 72, 73: step of the inventive manufacturing method
- 74, 75, 76: step of the inventive manufacturing method
- 77, 78, 79: step of the inventive manufacturing method
- 80: step of the inventive manufacturing method

## Claims

1. A lead connector assembly for a medical device, the lead connector assembly having an open end and a closed end and comprising
one or more electrically conducting contact rings and, if applicable, spaced apart by at least one electrically insulating and/or sealing ring forming a ring stack, wherein each contact ring comprises an electrically conducting pin protruding from the respective contact ring, and an electrically insulating end ring accommodated at the end of the ring stack at the open end of the lead connector assembly, wherein the inner surface of each of the one or more contact rings, of each of the at least one electrically insulating and/or sealing ring and of the end ring define a lead aperture starting at the open end of the lead connector assembly,
**characterized by** at least two connector housing parts which enclose the one or more contact rings, the electrically insulating and/or sealing ring and the end ring and forming an outer surface of the lead connector assembly, wherein the connector housing parts are joined such that a hermetic seal is provided between the lead aperture and the outer surface of the lead connector assembly, wherein the electrically conducting pin of each contact ring projects through at least one of the joined connector housing parts and from the outer housing of the lead connector assembly.

2. The lead connector assembly of claim 1, wherein at least one electrically insulating layer is accommodated between the outer surface of the one or more contact rings and the at least two connector housing parts.

3. The lead connector assembly of any of the previous claims, wherein the one or more contact ring forms a spring contact, preferably a garter spring contact.

4. The lead connector assembly of any of the previous claims, wherein an insulating bar is provided forming one of the at least two connector housing parts enclosing the end of each pin located opposite the respective contact ring.

5. The lead connector assembly of any of the previous claims, wherein a set screw block is provided, which is accommodated at the end of the ring stack located at the closed end of the lead connector assembly and enclosed by the at least two connector housing parts.

6. Medical device comprising a power source connected with an electronic circuit having a circuit board, further comprising at least one lead connector assembly according to any of the previous claims, wherein each pin projecting from the outer surface of each lead connector assembly is directly electrically connected to a terminal of the electronic circuit.

7. Medical device according to claim 6, wherein each terminal for connection with the respective pin of the lead connector assembly is provided at a flexible arm of the circuit board.

8. Medical device according to any of the claims 6 to 7, wherein a device housing is provided enclosing the power source, the electronic circuit and the at least one lead connector assembly.

9. Method of manufacturing the lead connector assembly of one of the claims 1 to 5, comprising the following steps:
a) inserting the one or more electrically conducting contact rings with their respective pin into an according opening in the at least one first connector housing part,
b) hermetically sealed joining the contact rings with the at least one first connector housing part,
c) accommodating at least one electrically insulating and/or sealing ring between two contact rings, if applicable, and accommodating an end ring at the end of the ring stack at the open end of the lead connector assembly, and
d) hermetically sealed joining of at least a second connector housing part to the first connector housing part.

10. Method according to claim 9, wherein prior step d) in step d1) at least one electrically insulating layer is accommodated between the outer surface of the one or more contact rings and the inner surface of the at least one first connector housing part, for example by rotating two halves of a sleeve into the respective opening or by injection molding or by casting.

11. Method according to any of claims 9 or 10, wherein prior to step d) and d1) a set screw block is accommodated at the end of the ring stack located at the closed end of the lead connector assembly.

12. Method of manufacturing the medical device of any of the claims 6 to 8 comprising the steps of the method of manufacturing the lead connector assembly of any of the claims 9 to 11 and the additional steps
e) directly electrically connecting each pin projecting from the outer surface of each lead connector assembly to a terminal of the electronic circuit and
f) enclosing the power source, the electronic circuit and the at least one lead connector assembly with a device housing.
